# FASCICULE DE BREVET EUROPEEN

(11) **EP 3 835 405 B1**
(45) Date de publication et mention de la délivrance du brevet: **27.07.2022**
(21) Numéro de dépôt: 20209604.6
(22) Date de dépôt: 24.11.2020
(51) Int. Cl.: C12M 1/107, C10L 3/10, C12M 1/00

(54) **DIGESTEUR COMPRENANT UNE PAROI INTERNE POREUSE**
FAULBEHÄLTER, DER EINE PORÖSE INNENWAND UMFASST
DIGESTER COMPRISING A POROUS INTERNAL WALL

(30) Priorité: 11.12.2019 FR 1914170
(43) Date de publication de la demande: 16.06.2021
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BERTRANDIAS, Aude, 78350 Les Loges-En-Josas (FR); FRIMAT, David, 78350 Les Loges-En-Josas (FR); TRUEBA, Antonio, 78350 Les Loges-En-Josas (FR); SEIWERT, Jacopo, 78350 Les Loges-En-Josas (FR); OLLIER, Jérémy, 38360 Sassenage (FR); VALENTIN, Solène, 38360 Sassenage (FR)
(74) Mandataire: Air Liquide

(56) Documents cités:
- EP-A1- 1 970 434
- WO-A1-2014/009575
- CN-A- 102 174 378
- DE-A1-102014 222 703

## Description

La présente invention est relative à une installation et un procédé pour la production de biogaz au moins partiellement désulfuré.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, et dont les conditions sont contrôlées, appelé méthaniseur ou digesteur, puis dans un post-digesteur, similaire au digesteur et permettant de pousser plus loin la réaction de méthanisation.

On appellera biomasse tout groupement de matières organiques pouvant se transformer en énergie à travers ce processus de méthanisation, par exemple les boues de station d'épuration, fumiers/lisiers, résidus agricoles, déchets alimentaires...

Le digesteur, c'est-à-dire le réacteur dédié à la méthanisation de la biomasse, est une cuve fermée, chauffée ou non (opération à une température fixée, entre la température ambiante et 55°C) et dont le contenu constitué de la biomasse est brassé, en continu ou séquentiel. Les conditions dans le digesteur sont anaérobies et le biogaz généré se retrouve dans l'espace de tête du digesteur (ciel gazeux), où il est prélevé. Les post-digesteurs sont similaires aux digesteurs.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH₄) et du dioxyde de carbone (CO₂) dans des proportions variables en fonction du mode d'obtention et du substrat mais peut également contenir, en moindres proportions de l'eau, de l'azote, de l'hydrogène sulfuré (H₂S), de l'oxygène, ainsi que des composés organiques autres, à l'état de traces, dont le H2S, entre 10 et 50,000 ppmv.

Selon les matières organiques dégradées et les techniques utilisées, les proportions des composants diffèrent, mais en moyenne le biogaz comporte, sur gaz sec, de 30 à 75% de méthane, de 15 à 60% de CO₂, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au coeur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (bioGNL)...

Selon la composition de la biomasse, le biogaz produit lors de la digestion contient du sulfure d'hydrogène (H₂S) dans des teneurs comprises entre 50 et 50 000 ppm.

Quelle que soit la destination finale de valorisation du biogaz, il s'avère indispensable d'éliminer le sulfure d'hydrogène qui est une impureté toxique et corrosive. De plus, si l'utilisation du biogaz consiste à l'épurer pour injecter du biométhane dans le réseau de gaz naturel, des spécifications strictes limitent la quantité de H₂S autorisée.

Plusieurs méthodes d'élimination du H₂S existent et sont plus ou moins répandues (lits de charbon actif, ajout de composés de fer, absorption physique ou chimique, lavages à l'eau, biofiltres...). L'élimination se fait principalement par adsorption sur lit de charbon actif, à l'extérieur du digesteur. Dans un nombre croissant de digesteurs, l'abattement de l'H2S se fait aussi en partie par injection d'air/air enrichi/O₂ dans le ciel gazeux du digesteur, ce qui constitue une solution in situ. Avec une injection dans le ciel gazeux à faible dose, du soufre solide est formé à partir du H₂S et O₂ (eq. (1)), réalisée par des bactéries sulfo-oxydantes e.g. Thiobacillus. A haute dose d'O₂ injecté, le milieu est acidifié (eq. (2)). La réaction (1) est donc ciblée.

H₂S + 0.5 O₂ → S + H₂O (1)

H₂S + 2 O₂ →SO₄ ²⁻ + 2 H + (2)

Les quantités d'injection d'O₂ nécessaires en pratiques sont différentes de celles attendues par la stoechiométrie de l'éq. (1): des doses de 0.3 - 3% d'O₂ par rapport au biogaz généré sont le plus souvent recommandées, avec des doses jusqu'à 12% qui sont parfois évoquées. Aujourd'hui, l'injection d'air/air enrichi/O₂ in situ n'est pas optimisée et les lits de charbon actif doivent donc être maintenus pour éliminer la totalité du H₂S. La demande CN 102174378 divulgue une installation de production de biogaz avec une enceinte pour la biomasse, un moyen d'introduction d'un gaz d'oxydation et une structure stratifiée plate avec des mailles fixées horizontalement dans l'enceinte.

Partant de là, un problème qui se pose est de fournir une installation améliorée favorisant l'élimination plus poussée de l'H₂S.

Une solution de la présente invention est une installation de production de biogaz au moins partiellement désulfuré comprenant un digesteur et/ou un post-digesteur de biomasse, le digesteur et/ou post-digesteur comprenant :
- Une enceinte comprenant la biomasse et le ciel gazeux, et
- Un moyen d'introduction d'un gaz d'oxydation,
caractérisée en ce que la portion de la paroi interne de l'enceinte située au niveau du ciel gazeux est au moins en partie poreuse.

Par « ciel gazeux » on entend l'espace dans le digesteur ou post-digesteur contenant du gaz (par opposition à l'espace qui contient le liquide).

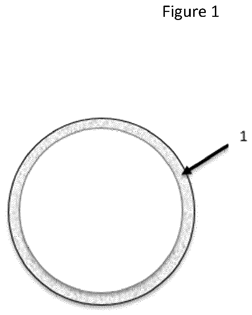

[Fig. 1] La figure 1 est une coupe schématique horizontale de l'enceinte du digesteur. Notons que la coupe est faite dans la portion de la paroi interne de l'enceinte située au niveau du ciel gazeux. Elle met en avant la partie poreuse 1.

Les réactions entre l'O₂ et l'H₂S se produisent sur une surface (pas dans la phase gazeuse ou liquide du digesteur). Les micro-organismes nécessaires à la réaction (bactéries sulfo-oxydantes telles que Thiobacillus ) sont dans la phase liquide et les réactifs sont en phase gazeuse. Une surface hydrophile est nécessaire pour mettre en contact la phase liquide, les micro-organismes et la phase gazeuse, les réactifs. Ces surfaces sont principalement les parois internes dans le ciel gazeux, l'interface gaz/liquide du digesteur ou post-digesteur ainsi que toute autre surface à disposition dans le ciel gazeux e.g. filets. Si les surfaces disponibles sont insuffisantes, les réactions d'abattement d'H₂S ne se produisent pas suffisamment voire pas du tout.

Autrement dit la solution selon l'invention est d'augmenter la surface de réaction entre la phase liquide et la phase gazeuse en ajoutant une ou plusieurs parties poreuses à la surface de la paroi interne de l'enceinte. Cette addition de masse poreuse permet d'augmenter la taille du support réactionnel et favorise ainsi l'élimination plus poussée du H₂S.

Selon le cas l'installation selon l'invention peut présenter une ou plusieurs des caractéristiques ci-dessous :
- la porosité de la portion de la paroi interne de l'enceinte située au niveau du ciel gazeux est comprise entre 75 et 95% du volume.
- la portion de la paroi interne de l'enceinte située au niveau du ciel gazeux comprend des sous-portions de paroi présentant une surface poreuse.
- la portion de la paroi interne de l'enceinte située au niveau du ciel gazeux est divisée en une première paroi et une deuxième paroi, les deux parois étant accolées, et la deuxième paroi étant en regard de l'intérieur de l'enceinte et présentant au moins une partie de sa surface poreuse.
- la partie poreuse de la portion de la paroi interne de l'enceinte située au niveau du ciel gazeux est composée d'un matériau qui est résistant à une atmosphère humide et corrosive, par exemple certains aciers inox, PEEK, PTFE.
- l'installation comprend un moyen de brassage favorisant un transfert du gaz d'oxydation vers la paroi interne de l'enceinte.

La présente invention a également pour objet un procédé de production de biogaz au moins partiellement désulfuré mettant en oeuvre une installation selon l'invention, comprenant :
- L'injection de biomasse dans le digesteur ;
- L'injection d'un gaz d'oxydation en tête du digesteur ; et
- Le brassage de la biomasse.

De préférence, le brassage de la biomasse est réalisé de manière à favoriser un transfert du gaz d'oxydation vers la paroi interne de l'enceinte.

Notons que le gaz d'oxydation pourra être de l'oxygène ou de l'air ou de l'air enrichi. Par air enrichi on entend de l'air présentant une teneur en oxygène plus élevée que la teneur en oxygène habituellement comprise dans l'air.

A l'intérieur du digesteur, lorsque le sulfure d'hydrogène réagit avec l'oxygène, le soufre se fixe sur la paroi interne de l'enceinte du digesteur. Au bout d'un certain temps, le soufre solide généré tombe dans le digestat et est évacué avec celui-ci.

La solution selon l'invention permet d'obtenir un flux de biogaz comprenant moins de 200 ppm de sulfure d'hydrogène.

L'invention permet de réduire le coût d'épuration du sulfure d'hydrogène du biogaz de façon efficace, en augmentant la réactivité de l'oxygène déjà injecté avec les produits soufrés grâce à la création d'une surface réactionnelle additionnelle sur les parois internes du digesteur au niveau du ciel gazeux, sans besoin complexe en ingénierie.

## Revendications

1. Installation de production de biogaz au moins partiellement désulfuré comprenant un digesteur et/ou un post-digesteur de biomasse, le digesteur et/ou le post-digesteur comprenant :
- Une enceinte comprenant la biomasse et le ciel gazeux, et
- Un moyen d'introduction d'un gaz d'oxydation,
**caractérisée en ce que** la portion de la paroi interne de l'enceinte située au niveau du ciel gazeux est au moins en partie poreuse.

2. Installation selon la revendication 1, **caractérisée en ce que** la porosité de la portion de la paroi interne de l'enceinte située au niveau du ciel gazeux est comprise entre 75 et 95% du volume.

3. Installation selon l'une des revendications 1 ou 2, **caractérisée en ce que** la portion de la paroi interne de l'enceinte située au niveau du ciel gazeux comprend des sous-portions de paroi présentant une surface poreuse.

4. Installation selon l'une des revendications 1 à 3, **caractérisée en ce que** la portion de la paroi interne de l'enceinte située au niveau du ciel gazeux est divisée en une première paroi et une deuxième paroi, les deux parois étant accolées, et la deuxième paroi étant en regard de l'intérieur de l'enceinte et présentant au moins une partie de sa surface poreuse.

5. Installation selon l'une des revendications 1 à 4, **caractérisée en ce que** la partie poreuse de la portion de la paroi interne de l'enceinte située au niveau du ciel gazeux est composée d'un matériau qui est résistant à une atmosphère humide et corrosive.

6. Installation selon l'une des revendications 1 à 4, **caractérisée en ce qu'**elle comprend un moyen de brassage favorisant un transfert du gaz d'oxydation vers la paroi interne de l'enceinte.

7. Procédé de production de biogaz au moins partiellement désulfuré mettant en oeuvre une installation selon l'une des revendications 1 à 6, comprenant :
- L'injection de biomasse dans le digesteur ;
- L'injection d'un gaz d'oxydation en tête du digesteur ; et
- Le brassage de la biomasse.

8. Procédé selon la revendication 7, **caractérisé en ce que** le brassage de la biomasse est réalisée de manière à favoriser un transfert du gaz d'oxydation vers la paroi interne de l'enceinte.

9. Procédé selon l'une des revendications 7 ou 8, **caractérisé en ce que** le gaz d'oxydation est de l'oxygène ou de l'air ou de l'air enrichi.

## Patentansprüche

1. Anlage zur Produktion von zumindest teilweise entschwefeltem Biogas, umfassend einen Biomasse-Fermenter und/oder -Nachgärer, wobei der Fermenter und/oder der Nachgärer Folgendes umfasst:
- einen Behälter, der die Biomasse und den Gasraum umfasst, und
- ein Mittel zum Einbringen eines Oxidationsgases, **dadurch gekennzeichnet, dass** der Abschnitt der Innenwand des Behälters, der sich in Höhe des Gasraums befindet, zumindest zum Teil porös ist.

2. Anlage nach Anspruch 1, **dadurch gekennzeichnet, dass** die Porosität des Abschnitts der Innenwand des Behälters, der sich in Höhe des Gasraums befindet, zwischen 75 und 95 Volumen-% beträgt.

3. Anlage nach einem der Ansprüche 1 oder 2, **dadurch gekennzeichnet, dass** der Abschnitt der Innenwand des Behälters, der sich in Höhe des Gasraums befindet, Wandteilabschnitte umfasst, die eine poröse Oberfläche aufweisen.

4. Anlage nach einem der Ansprüche 1 bis 3, **dadurch gekennzeichnet, dass** der Abschnitt der Innenwand des Behälters, der sich in Höhe des Gasraums befindet, in eine erste Wand und eine zweite Wand unterteilt ist, wobei die beiden Wände aneinander liegen und die zweite Wand zum Inneren des Behälters hin zeigt und mindestens einen Teil ihrer porösen Oberfläche zeigt.

5. Anlage nach einem der Ansprüche 1 bis 4, **dadurch gekennzeichnet, dass** der poröse Teil des Abschnitts der Innenwand des Behälters, der sich in Höhe des Gasraums befindet, aus einem Material besteht, das gegenüber einer feuchten und korrosiven Atmosphäre beständig ist.

6. Anlage nach einem der Ansprüche 1 bis 4, **dadurch gekennzeichnet, dass** sie ein Rührmittel umfasst, das den Transport des Oxidationsgases zur Innenwand des Behälters fördert.

7. Verfahren zur Herstellung von zumindest teilweise entschwefeltem Biogas unter Verwendung einer Anlage nach einem der Ansprüche 1 bis 6, umfassend:
- Einspritzen von Biomasse in den Fermenter;
- Einleiten eines Oxidationsgases im oberen Abschnitt des Fermenters und
- Rühren der Biomasse.

8. Verfahren nach Anspruch 7, **dadurch gekennzeichnet, dass** das Rühren der Biomasse derart durchgeführt wird, dass der Transport des Oxidationsgases zur Innenwand des Behälters gefördert wird.

9. Verfahren nach einem der Ansprüche 7 oder 8, **dadurch gekennzeichnet, dass** das Oxidationsgas Sauerstoff oder Luft oder angereicherte Luft ist.

## Claims

1. Plant for producing at least partially desulfurized biogas, comprising a biomass digester and/or post-digester, the digester and/or post-digester comprising:
- a chamber comprising the biomass and the gas space, and
- a means for introducing an oxidizing gas, **characterized in that** the portion of the inner wall of the chamber situated at the level of the gas space is at least partly porous.

2. Plant according to Claim 1, **characterized in that** the porosity of the portion of the inner wall of the chamber situated at the level of the gas space is between 75% and 95% of the volume.

3. Plant according to either of Claims 1 and 2, **characterized in that** the portion of the inner wall of the chamber situated at the level of the gas space comprises wall sub-portions having a porous surface.

4. Plant according to any of Claims 1 to 3, **characterized in that** the portion of the inner wall of the chamber situated at the level of the gas space is divided into a first wall and a second wall, the two walls being side by side, and the second wall being opposite the interior of the chamber and having at least part of its surface porous.

5. Plant according to any of Claims 1 to 4, **characterized in that** the porous part of the portion of the inner wall of the chamber situated at the level of the gas space is composed of a material which is resistant to a humid and corrosive atmosphere.

6. Plant according to any of Claims 1 to 4, **characterized in that** it comprises a mixing means which promotes transfer of the oxidizing gas to the inner wall of the chamber.

7. Process for producing at least partially desulfurized biogas, using a plant according to any of Claims 1 to 6, which comprises:
- injecting biomass into the digester;
- injecting an oxidizing gas at the top of the digester; and
- mixing the biomass.

8. Process according to Claim 7, **characterized in that** the biomass is mixed so as to promote transfer of the oxidizing gas to the inner wall of the chamber.

9. Process according to either of Claims 7 and 8, **characterized in that** the oxidizing gas is oxygen or air or enriched air.
